Europäisches Patentamt
European Patent Office
Office européen des brevets

(19)

(11) Publication number: **0 158 265 A2**

(12) **EUROPEAN PATENT APPLICATION**

(21) Application number: 85103968.5

(22) Date of filing: 02.04.85

(51) Int. Cl.[4]: C 07 D 451/04, A 61 K 31/435

(30) Priority: 13.04.84 GB 8409699
22.02.85 GB 8504608

(43) Date of publication of application: 16.10.85 Bulletin 85/42

(84) Designated Contracting States: BE CH DE FR GB IT LI NL SE

(71) Applicant: BEECHAM GROUP PLC
Beecham House Great West Road
Brentford Middlesex TW8 9BD(GB)

(72) Inventor: Wootton, Gordon
51 Parkway
Sawbridgeworth Hertfordshire(GB)

(72) Inventor: Sanger, Gareth John
15 Hedgerows
Sawbridgeworth Hertfordshire CM21 9BQ(GB)

(74) Representative: Jones, Pauline et al,
Beecham Pharmaceuticals Patents & Trade Marks Dept.
Great Burgh Yew Tree Bottom Road
Epsom Surrey KT18 5XQ(GB)

(54) **Benzamide and anilide derivatives.**

(57) Compounds of formula (I), or a pharmaceutically acceptable salt thereof, or a pharmaceutically acceptable solvate of either of the foregoing:

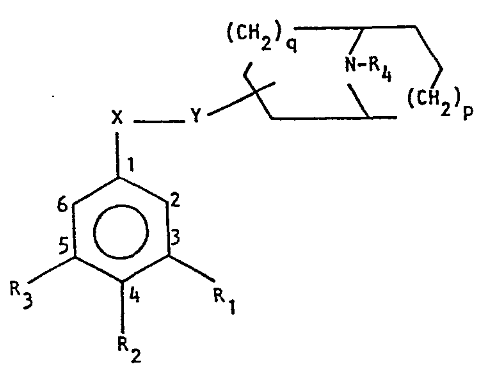

(I)

wherein

one of X and Y is CO and the other is NH;

p and q each independently are 0 to 2;

$R_1$, $R_2$ and $R_3$ are each independently hydrogen, $C_{1-6}$ alkyl, hydroxy, $C_{1-6}$ alkoxy, $C_{1-6}$ alkylthio or halo; or any two on adjacent carbon atoms together are $C_{1-2}$ alkylenedioxy and the third is hydrogen, $C_{1-6}$ alkyl, $C_{1-6}$ alkoxy or halo; and

$R_4$ is $C_{1-7}$ alkyl, $C_{3-8}$ cycloalkyl, $C_{3-8}$ cycloalkyl-$C_{1-2}$ alkyl, or a group $(CH_2)_tR_5$ where t is 1 or 2 and $R_5$ is thienyl or furyl optionally substituted by one or two substituents selected from $C_{1-6}$ alkyl, $C_{1-6}$ alkoxy, trifluoromethyl or halogen, or is phenyl optionally substituted by one or two substituents selected from $C_{1-4}$ alkoxy, trifluoromethyl, halogen, nitro, carboxy, esterified carboxy, and $C_{1-4}$ alkyl optionally substituted by hydroxy, $C_{1-4}$ alkoxy, carboxy, esterified carboxy or in vivo hydrolysable acyloxy, having 5-HT antagonist activity, a process for their preparation and their use as pharmaceuticals.

EP 0 158 265 A2

BENZAMIDE AND ANILIDE DERIVATIVES

This invention relates to novel compounds having useful pharmacological properties, to pharmaceutical compositions containing them, to a process and intermediates for their preparation, and to the use of the compounds.

US Patent No. 4,273,778, European Patent 13138 and European Patent Publications 31219 and 41817 disclose benzamides having an azabicyclic side chain and possessing dopamine antagonist activity.

A class of novel, structurally distinct compounds has now been discovered. These compounds have 5-HT antagonist activity.

Accordingly, the present invention provides a compound of formula (I), or a pharmaceutically acceptable salt thereof, or a pharmaceutically acceptable solvate of either of the foregoing:

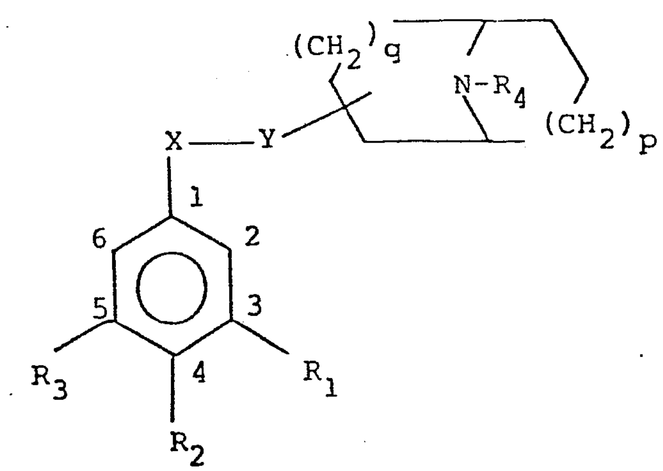

(I)

wherein

one of X and Y is CO and the other is NH;

p and q each independently are 0 to 2;

$R_1$, $R_2$ and $R_3$ are each independently hydrogen, $C_{1-6}$ alkyl, hydroxy, $C_{1-6}$ alkoxy, $C_{1-6}$ alkylthio or halo; or any two on adjacent carbon atoms together are $C_{1-2}$ alkylenedioxy and the third is hydrogen, $C_{1-6}$ alkyl, $C_{1-6}$ alkoxy or halo; and

$R_4$ is $C_{1-7}$ alkyl, $C_{3-8}$ cycloalkyl, $C_{3-8}$ cycloalkyl-$C_{1-2}$ alkyl, or a group $(CH_2)_tR_5$ where t is 1 or 2 and $R_5$ is thienyl or furyl optionally substituted by one or two substituents selected from $C_{1-6}$ alkyl, $C_{1-6}$ alkoxy, trifluoromethyl or halogen, or is phenyl optionally substituted by one or two substituents selected from $C_{1-4}$ alkoxy, trifluoromethyl, halogen, nitro, carboxy, esterified carboxy, and $C_{1-4}$ alkyl optionally substituted by hydroxy, $C_{1-4}$ alkoxy, carboxy, esterified carboxy or in vivo hydrolysable acyloxy.

Preferably X is CO and Y is NH.

p may be 0 or 1, preferably 0, and q may be 0 or 1, in particular 1.

The group Y and the heterobicycle nitrogen atom may be separated by 2 or 3 carbon atoms, preferably 3.

The X,Y moiety may be in an equatorial or axial orientation to the heterobicycle ring.

Examples of $R_1$ include halo such as chloro, and $C_{1-6}$ alkoxy, such as methoxy. Preferably $R_1$ is chloro.

Examples of $R_2$ include hydrogen, halo, such as chloro, hydroxy and $C_{1-6}$ alkoxy, such as methoxy. Preferably $R_2$is hydrogen or chloro.

Examples of $R_3$ include hydrogen, halo such as chloro, and $C_{1-6}$ alkoxy, such as methoxy. Preferably $R_3$ is hydrogen or chloro.

Examples of $R_4$, when $-(CH_2)_tR_5$, are those wherein t is 1. $R_5$ may be 2- or 3-thienyl or preferably is phenyl optionally substituted by one of $C_{1-4}$ alkoxy, trifluoromethyl, halogen, carboxy, esterified carboxy or $C_{1-4}$ alkyl optionally substituted by hydroxy, $C_{1-4}$ alkoxy, carboxy, esterified carboxy and in vivo hydrolysable acyloxy.

Examples of $R_4$ when $C_{1-7}$ alkyl include methyl and ethyl. Within $C_{1-7}$ alkyl, $C_{4-7}$ alkyl are of interest, especially those of the form $(CH_2)_uR_6$ wherein u is 1 or 2 and $R_6$ is a secondary or tertiary $C_{3-6}$ alkyl group. Examples of $C_{4-7}$ alkyl include 2,2-dimethylpropyl. $R_4$ is preferably $C_{1-7}$ alkyl, most preferably methyl.

The pharmaceutically acceptable salts of the compounds of the formula (I) include acid addition salts with conventional acids such as hydrochloric, hydrobromic, boric, phosphoric, sulphuric acids and pharmaceutically acceptable organic acids such as acetic, tartaric, maleic, citric, succinic, benzoic, ascorbic, methanesulphonic, α-keto glutaric, α-glycerophosphoric, and glucose-1-phosphoric acids.

The pharmaceutically acceptable salts of the compounds of the formula (I) are usually acid addition salts with acids such as hydrochloric, hydrobromic, phosphoric, sulphuric, citric, tartaric, lactic and acetic acid.

Preferably the acid addition salt is the hydrochloride salt.

Examples of quaternary derivatives of the compounds of formula (I) include the compounds quaternised by compounds such as $R_8$-Q wherein $R_8$ is $C_{1-6}$ alkyl, phenyl-$C_{1-6}$ alkyl or $C_{5-7}$ cycloalkyl, and Q is a radical corresponding to an anion of an acid. Suitable examples of $R_8$ include methyl, ethyl and n- and iso-propyl; and benzyl and phenethyl. Suitable examples of Q include halide such as chloride, bromide and iodide.

The compounds of formula (I) may also form pharmaceutically acceptable N-oxides.

The compounds of the formula (I), their pharmaceutically acceptable salts, quaternary derivatives and N-oxides may also form pharmaceutically acceptable solvates.

It will of course be realised that the compounds of the formula (I) have chiral or prochiral centres, and thus are capable of existing in a number of stereoisomeric forms, including enantiomers. The invention extends to each of these stereoisomeric forms, and to mixtures thereof (including racemates). The different stereoisomeric forms may be separated or resolved one from the other by the usual methods, or any given isomer may be obtained by stereospecific or asymmetric synthesis.

A group of compounds of formula (I) is of formula (II):

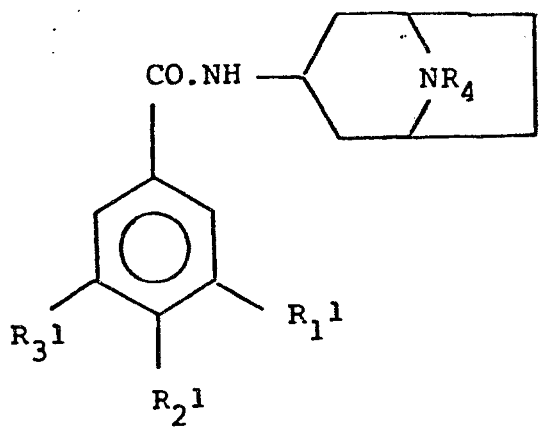

(II)

wherein

$R_1{}^1$ is halo or $C_{1-6}$ alkoxy;

$R_2{}^1$ is hydrogen or $C_{1-6}$ alkoxy;

$R_3{}^1$ is halo or $C_{1-6}$ alkoxy; and

$R_4$ is as defined in formula (I).

Examples and preferred values of the variables are as so described for corresponding variables under formula (I).

The invention also provides a process for the preparation of a compound of formula (I), or a pharmaceutically acceptable salt thereof, or a pharmaceutically acceptable solvate of any of the foregoing, which process comprises reacting a compound of formula (III):

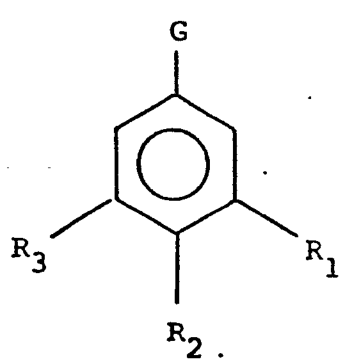

(III)

with a compound of formula (IV):

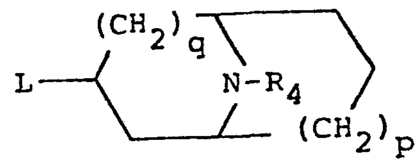

(IV)

wherein

one of G and L is $COQ_1$ where $Q_1$ is a group displaceable by a nucleophile, and the other is $NH_2$; and

the remaining variables are as hereinbefore defined; and

thereafter optionally converting any $R_1$, $R_2$, $R_3$ or $R_4$ group to another $R_1$, $R_2$, $R_3$ or $R_4$ group respectively, and optionally forming a pharmaceutically acceptable salt of the resultant compound of formula (I).

Examples of groups $Q_1$ displaceable by a nucleophile include halogen such as chloro and bromo, hydroxy, carboxylic acyloxy such as $C_{1-4}$ alkanoyloxy or $C_{1-4}$ alkoxycarbonyloxy and activated hydrocarbyloxy such as pentachlorophenoxy.

If a group $Q_1$ is a halide, then the reaction is preferably carried out at non-extreme temperatures in an inert non-hydroxylic solvent, such as benzene, dichloromethane, toluene, diethyl ether, THF or DMF. It is also preferably carried out in the presence of an acid acceptor, such as an organic base, in particular a tertiary amine, such as triethylamine, trimethylamine, pyridine or picoline, some of which can also function as the solvent. Alternatively, the acid acceptor can

be inorganic, such as calcium carbonate, sodium carbonate or potassium carbonate. Temperatures of 0°-100°C, in particular 10-80°C are suitable.

If a group $Q_1$ is hydroxy, then the reaction is generally carried out in an inert non-hydroxylic solvent, such as dichloromethane, THF or DMF optionally in the presence of a dehydrating catalyst, such as a carbodiimide, for example dicyclohexylcarbodiimide. When Y is CO the compound of formula (IV) is preferably in the form of an acid addition salt, such as the hydrohalide, for example the hydrochloride. The reaction may be carried out at any non-extreme temperature, such as -10 to 100°C, for example, 0 to 80°C. Generally, higher reaction temperatures are employed with less active compounds whereas lower temperatures are employed with the more active compounds.

If a group $Q_1$ is carboxylic acyloxy, then the reaction is preferably carried in substantially the same manner as the reaction when $Q_1$ is halide. Suitable examples of acyloxy leaving groups include $C_{1-4}$ alkanoyloxy and $C_{1-4}$ alkoxycarbonyloxy, in which case the reaction is preferably carried out in an inert solvent, such as methylene chloride, at a non-extreme temperature for example ambient temperatures in the presence of an acid acceptor, such as triethylamine. $C_{1-4}$ alkoxycarbonyloxy leaving groups may be generated in situ by treatment of the corresponding compound wherein Q1 is hydroxy with a $C_{1-4}$ alkyl chloroformate.

If a group $Q_1$ is activated hydrocarbyloxy then the reaction is preferably carried out in an inert polar solvent, such as dimethylformamide. It is also

preferred that the activated hydrocarbyloxy group is a pentachlorophenyl ester and that the reaction is carried out at ambient temperature.

Pharmaceutically acceptable salts of the compounds of this invention may be formed conventionally.

The salts may be formed for example by reaction of the base compound of formula (I) with a pharmaceutically acceptable organic or inorganic acid.

It will be apparent that compounds of the formula (I) containing an $R_1$, $R_2$, $R_3$ or $R_4$ group which is convertible to another $R_1$, $R_2$, $R_3$ or $R_4$ group are useful novel intermediates. A number of such conversions is possible not only for the end compounds of formula (I), but also for their intermediates.

For example an $R_1$, $R_2$ or $R_3$ hydrogen substituent is convertible to a halogen substituent by halogenation with a conventional halogenating agent.

$R_4$ optionally substituted benzyl as hereinbefore defined may be replaced by other $R_4$. Such $R_4$ benzyl groups may, for example, be removed, when $R_1$, $R_2$ or $R_3$ is not halogen, by conventional transition metal catalysed hydrogenolysis to give compounds of the formula (X):

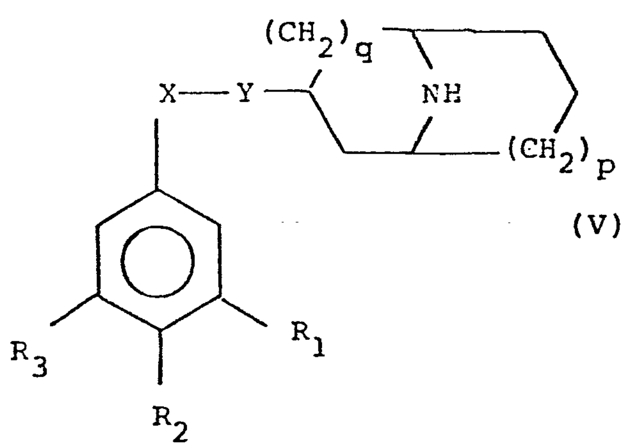

(V)

wherein the variables are as defined in formula (I).

This invention also provides a second process for the preparation of a compound of the formula (I) which comprises N-alkylating a compound of formula (V), and optionally forming a pharmaceutically acceptable salt, quaternary derivative or N-oxide of the resulting compound of the formula (I).

In this second process of the invention 'N-alkylation' comprises the substitution of the N-atom depicted in formula (V) by any group $R_4$ as hereinbefore defined. This may be achieved by reaction of the compound of formula (V) with a compound $R_4Q_2$ wherein $R_4$ is as hereinbefore defined and $Q_2$ is a leaving group.

Suitable values for $Q_2$ include groups displaced by nucleophiles such as Cl, Br, I, $OSO_2CH_3$ or $OSO_2C_6H_4pCH_3$.

Favoured values for $Q_2$ include Cl, Br and I.

The reaction may be carried out under conventional alkylation conditions for example in an inert solvent such as dimethylformamide in the presence of an acid acceptor such as potassium carbonate. Generally the reaction is carried out at non-extreme temperature such as at ambient or slightly above.

Alternatively, 'N-alkylation' may be effected under conventional reductive alkylation conditions when the final group $R_4$ in the compound of formula (I) contains a methylene group adjacent to the N-atom in the bicycle.

Interconverting $R_4$ in the compound of the formula (IV) before coupling with the compound of the formula (III) is also possible. Such interconversions are effected conveniently under the above conditions. It is desirable to protect any amine function in the compound of formula (IV) with a group readily removable by acidolysis such as a $C_{2-7}$ alkanoyl group, before $R_4$ interconversion.

The same reductive alkylation route may be applied for the preparation of relevant intermediates of formula (IV), and reference to the preparation of such intermediates hereinafter should be read to include the preparation of the corresponding N-acyl intermediates.

The substituents in the phenyl ring when $R_4$ is phenyl $C_{1-2}$ alkyl in a compound of formula (I), in particular the substituted $C_{1-4}$ alkyl substituents, are interconvertible. A number of such interconversions are possible not only for the end compounds of formula (I), but also for their intermediates as follows:

(i) a carboxy $C_{1-4}$ alkyl substituent is convertible to an esterified carboxy $C_{1-4}$ alkyl substituent by esterification;

(ii) an esterified carboxy $C_{1-4}$ alkyl substituent is convertible to a carboxy $C_{1-4}$ alkyl substituent by de-esterification;

(iii) an $C_{1-4}$ alkoxy $C_{1-4}$ alkyl substituent or an <u>in vivo</u> hydrolysable $C_{2-4}$ acyloxy $C_{1-4}$ alkyl substituent is convertible to an hydroxy $C_{1-4}$ alkyl substituent by de-etherification;

(iv) a hydroxy $C_{1-4}$ alkyl substituent is convertible to $C_{1-4}$ alkoxy $C_{1-4}$ alkyl by O-alkylation or to <u>in vivo</u> hydrolysable $C_{1-4}$ acyloxy $C_{1-4}$ alkyl by O-acylation.

Conversions (i) to (iv) are only exemplary and are not exhaustive of the possibilities.

In regard to (i) and (ii), the esterification and de-esterification reactions are carried out in conventional manner.

In regard to (iii), a $C_{1-4}$ alkoxy $C_{1-4}$ alkyl substituent is convertible to an hydroxy $C_{1-4}$ alkyl substituent by conventional methods, such as warming with aqueous hydrobromic acid or by treatment with pyridine hydrochloride, boron tribromide, boron triiodide or iodotrimethylsilane.

An <u>in vivo</u> hydrolysable $C_{2-4}$ acyloxy $C_{1-4}$ alkyl substituent is convertible to an hydroxy $C_{1-4}$ alkyl substituent by acid or base hydrolysis.

In regard to (iv), O-alkylation is carried out under conventional conditions in an inert solvent at a non-extreme temperature such as ambient temperature or slightly above or at reflux temperature. The $C_{1-4}$ alkylating agent has a leaving group that is readily displaceable by a nucleophile. Examples of leaving groups include halide, such as chloride, bromide or iodide, or labile acyloxy groups, such as mesyl and tosyl.

O-acylation is carried out under conventional conditions with an acylating agent which has an acyl group capable of forming an <u>in vivo</u> hydrolysable

acyloxy group and leaving group, such as halide, for example chloride and bromide, and hydroxy. When halide is the leaving group, the reaction is generally carried out in the presence of a base. When hydroxy is the leaving group, the reaction is generally carried out in the presence of a dehydrating agent, such as dicyclohexylcarbodiimide, in an inert solvent at non-extreme temperature, such as ambient temperature or slightly above, or reflux temperature.

Before carrying out any of these conversions, the effect, if any, on other substituents should be considered, and such reagents as are appropriate should be selected together with the adoption of such precautionary measures as are necessary. For example, O-alkylation and O-acylation may also produce N-alkylated and N-acylated products respectively unless the nitrogen atom(s) is (are) previously protected. This may be conveniently achieved by carrying out the alkylation or acylation reaction in a strong acid, such as trifluoroacetic acid, which protonates, and thereby protects, the nitrogen atom(s).

The compounds of formula (III) and (IV) are known or are preparable analogously to, or routinely from, known compounds, such as those described in European Patent Publication No.13138 and US Patent No. 4336259.

As mentioned hereinbefore the compounds of formula (I) exist in a number of stereoisomeric forms, in particular the moiety X,Y as hereinbefore defined can be in the axial or the equatorial orientation to the bicyclic ring system. A mixture of such isomers can be obtained by a non-stereospecific process and then the desired isomer separated conventionally from the mixture by, for example, chromatography.

Compounds of the formula (IV) wherein L is $NH_2$, or their ring N-acyl analogues as appropriate as discussed hereinbefore, may be prepared non-stereospecifically from known compounds by the processes illustrated in the US and European Patents mentioned hereinbefore.

The compounds of the present invention are 5HT antagonists and it is thus believed may generally be used in the treatment or prophylaxis of migraine, cluster headaches and trigeminal neuralgia.

The compounds of the present invention also have anti-emetic activity; in particular that of preventing vomiting and nausea associated with cancer chemotherapy.

The invention also provides a pharmaceutical composition comprising a compound of the present invention, in particular a compound of formula (I), or a pharmaceutically acceptable salt thereof, or a pharmaceutically acceptable solvate of either of the foregoing and a pharmaceutically acceptable carrier.

Such compositions are prepared by admixture and are suitably adapted for oral or parenteral administration, and as such may be in the form of tablets, capsules, oral liquid preparations, powders, granules, lozenges, reconstitutable powders, injectable and infusable solutions or suspensions or suppositories. Orally administrable compositions are preferred, since they are more convenient for general use.

Tablets and capsules for oral administration are usually presented in a unit dose, and contain conventional excipients such as binding agents,

fillers, diluents, tabletting agents, lubricants, disintegrants, colourants, flavourings, and wetting agents. The tablets may be coated according to well known methods in the art, for example with an enteric coating.

Suitable fillers for use include cellulose, mannitol, lactose and other similar agents. Suitable disintegrants include starch, polyvinylpolypyrrolidone and starch derivatives such as sodium starch glycollate. Suitable lubricants include, for example, magnesium stearate.

Suitable pharmaceutically acceptable wetting agents include sodium lauryl sulphate. Oral liquid preparations may be in the form of, for example, aqueous or oily suspensions, solutions, emulsions, syrups, or elixirs, or may be presented as a dry product for reconstitution with water or other suitable vehicle before use. Such liquid preparations may contain conventional additives such as suspending agents, for example sorbitol, syrup, methyl cellulose, gelatin, hydroxyethylcellulose, carboxymethylcellulose, aluminium stearate gel or hydrogenated edible fats, emulsifying agents, for example lecithin, sorbitan monooleate, or acacia; non-aqueous vehicles (which may include edible oils), for example, almond oil, fractionated coconut oil, oily esters such as esters of glycerine, propylene glycol, or ethyl alcohol; preservatives, for example methyl or propyl p-hydroxybenzoate or sorbic acid, and if desired conventional flavouring or colouring agents.

Oral liquid preparations are usually in the form of aqueous or oily suspensions, solutions, emulsions, syrups, or elixirs or are presented as a dry product

for reconstitution with water or other suitable vehicle before use. Such liquid preparations may contain conventional additives such as suspending agents, emulsifying agents, non-aqueous vehicles (which may include edible oils), preservatives, and flavouring or colouring agents.

The oral compositions may be prepared by conventional methods of blending, filling, tabletting or the like. Repeated blending operations may be used to distribute the active agent throughout those compositions employing large quantities of fillers. Such operations are, of course, conventional in the art.

For parenteral administration, fluid unit dose forms are prepared containing a compound of the present invention and a sterile vehicle. The compound, depending on the vehicle and the concentration, can be either suspended or dissolved. Parenteral solutions are normally prepared by dissolving the compound in a vehicle and filter sterilising before filling into a suitable vial or ampoule and sealing. Advantageously, adjuvants such as a local anaesthetic, preservatives and buffering agents are also dissolved in the vehicle. To enhance the stability, the composition can be frozen after filling into the vial and the water removed under vacuum.

Parenteral suspensions are prepared in substantially the same manner except that the compound is suspended in the vehicle instead of being dissolved and sterilised by exposure of ethylene oxide before suspending in the sterile vehicle. Advantageously, a surfactant or wetting agent is included in the composition to facilitate uniform distribution of the compound of the invention.

The invention further provides a method of treatment or prophylaxis of migraine, cluster headache, trigeminal neuralgia and/or emesis in mammals, such as humans, which comprises the administration of an effective amount of a compound of the formula (I) or a pharmaceutically acceptable salt thereof, or a pharmaceutically acceptable solvate of either of the foregoing.

An amount effective to treat the disorders hereinbefore described depends on the relative efficacies of the compounds of the invention, the nature and severity of the disorder being treated and the weight of the mammal. However, a unit dose for a 70kg adult will normally contain 0.5 to 1000mg for example 1 to 500mg, of the compound of the invention. Unit doses may be administered once or more than once a day, for example, 2, 3 or 4 times a day.

No adverse toxicological effects are indicated at any of the aforementioned dosage ranges.

The invention also provides a compound of formula (I) or a pharmaceutically acceptable salt thereof for use as an active therapeutic substance, in particular for use in the treatment of migraine, cluster headache, trigeminal neuralgia and/or emesis.

The following Examples illustrate the preparation of compounds of formula (I).

EXAMPLE 1

3,5-Dichloro-N-[3β-{8-(2,2-dimethylpropyl)-8-azabicyclo(3.2.1)octyl}]benzamide (1)

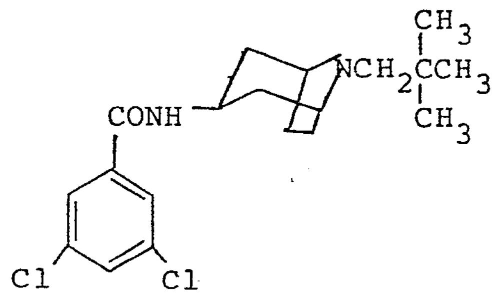

Ethyl chloroformate (0.6ml) in dichloromethane (15ml) was added dropwise to a stirred solution of 3,5-dichlorobenzoic acid (1.17g) and triethylamine (0.75ml) in dichloromethane, under nitrogen.

After 15 minutes, 3β-amino-8-(2,2-dimethylpropyl)-8-azabicyclo(3.2.1)octane (1.2g) in dichloromethane (15ml) was added dropwise and the mixture was stirred at room temperature, overnight.

10% Sodium hydroxide solution was added and the mixture was stirred for ½hr. The dichloromethane solution was separated, washed with brine, dried, and evaporated *in vacuo* to give a white solid (1.86g). This was chromatographed on silica gel (20:1) using ethylacetate/petrol (1:1) as eluant and the pure product was recrystallised from ethyl acetate to give the title compound (950mg) as glistening white needles. mp 207-8°C.

$C_{19}H_{26}N_2OCl_2$ required: C, 61.79; H, 7.1; N, 7.58%
found: C, 61.79; H, 7.13; N, 7.47%

<u>Examples 2 to 3</u>

In a similar manner, using 3β-amino-8-methyl-8-azabicyclo (3.2.1)octane, was obtained 3,5-dichloro-N-[3β-{8-methyl-8-azabicyclo(3.2.1)octyl}] benzamide. (2)

In a similar manner, using 3β-amino-8-benzyl-8-azabicyclo (3.2.1)octane, was obtained 3,5-dichloro-N-[3β-{8-benzyl-8-azabicyclo(3.2.1)octyl}] benzamide. (3)

EXAMPLE 4

3,4,5-Trimethoxy-N-[3β-{8-methyl-8-azabicyclo(3.2.1)octyl}] benzamide (4).

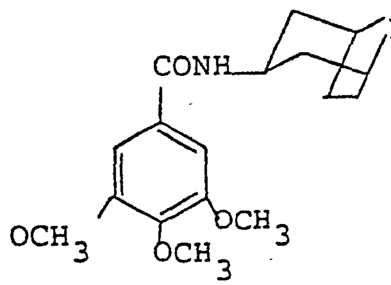

(4)

3β-amino-8-methyl-8-azabicyclo(3.2.1)octane (2g) in dichloromethane (20ml) was added dropwise to a stirred solution of 3,4,5-trimethoxybenzoylchloride (3.3g) and triethylamine (2ml) in dichloromethane (50ml), under nitrogen. The mixture was stirred at room temperature for 3hrs then 10% sodium hydroxide was added and the stirring was continued for a further ½hr. The organic phase was separated, washed with brine, dried and evaporated in vacuo to give a yellow solid (3.5g). This was chromatographed on 5% de-activated alumina (20:1) using dichloromethane as eluant to give 1.8g of slightly impure product. Recrystallisation from ethyl acetate gave the title compound (840mg).

m.s. $C_{18}H_{26}N_2O_4$ required 334.1892
found 334.1905

EXAMPLE 5



3,5-Dichloro-N-[3α-{8-methyl-8-azabicyclo(3.2.1)octyl}] benzamide (5)

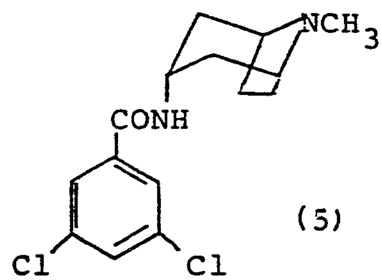

(5)

Ethyl chloroformate (2.76ml) in dichloromethane (30ml) was added dropwise to a stirred solution of 3,5-dichlorobenzoic acid (5.5g) and triethylamine (3.55ml) in dichloromethane (50ml), under nitrogen.

After 15 minutes, 3α-amino-8-methyl-8-azabicyclo (3.2.1)octane (4g) in dichloromethane (40ml) was added dropwise and the mixture was stirred at room temperature, overnight.

10% Sodium hydroxide solution was added the mixture was stirred for about ½hr. The dichloromethane solution was separated, washed with brine, dried ($Na_2SO_4$), and evaporated <u>in vacuo</u> to give a white solid (7g). This was chromatographed on silica gel (140g) using dichloromethane, 2% methanol in dichloromethane, 5% methanol in dichloromethane then 5% methanol in dichloromethane containing 0.5% ammonium hydroxide as eluants to give almost pure title compound (5.2g). Recrystallisation from ethyl acetate gave the title compound (2.7g) as white crystals. m.p. 158-9°C.

$C_{15}H_{18}N_2OCl_2$ required: C, 57.52; H, 5.79; N, 8.94%
found: C, 57.14; H, 5.88; N, 9.06%

EXAMPLE 6

3,4-Dichloro-N-[3β-{8-methyl-8-azabicyclo(3.2.1)octyl}] benzamide (6)

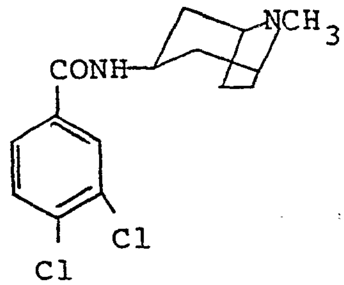

(6)

A mixture of 3,4-dichlorobenzoyl chloride (2.1g), triethylamine (1.23ml) and 3β-amino-8-methyl-8-azabicyclo(3.2.1)octane (1.4g) in dry dichloromethane (100ml) was stirred at room temperature, under nitrogen, overnight.

10% sodium hydroxide solution was added and the mixture was stirred for about ½hr. The dichloromethane solution was separated, washed with brine, dried ($Na_2SO_4$), and evaporated in vacuo to give a brown solid (2.57g). Recrystallisation from ethyl acetate gave the title compound as pale yellow crystals. m.p. 204.5-208.5°C.

| | | |
|---|---|---|
| $C_{15}H_{18}N_2OCl_2$ | required: | C, 57.52; H, 5.79; N, 8.94; Cl, 22.64% |
| | found: | C, 57.44; H, 5.72; N, 8.88; Cl, 22.57% |

0158265

## Pharmacology

### Antagonism of the von Bezold-Jarisch reflex

The compounds were evaluated for antagonism of the von Bezold-Jarisch reflex evoked by 5HT in the anaesthetised rat according to the following method:

Male rats (Beecham Pharmaceuticals), 250-350g, were anaesthetised with urethane (1.25g/kg intraperitoneally) and blood pressure and heart rate recorded as described by Fozard J.R. et al., J. Cardiovasc. Pharmacol. 2, 229-245 (1980). A submaximal dose of 5-HT (usually 6μg/kg) was given repeatedly by the intravenous route and changes in heart rate quantified. Examples were given intravenously and the concentration required to reduce the 5HT-evoked response to 50% of the control response ($ED_{50}$) was then determined.

The results obtained are shown in the following table.

| Compound No. | $ED_{50}$ mg/kg i.v. |
|---|---|
| (2) | 0.3 |
| (5) | 0.009 |

Claims

1. A compound of formula (I), or a pharmaceutically acceptable salt thereof, or a pharmaceutically acceptable solvate of either of the foregoing:

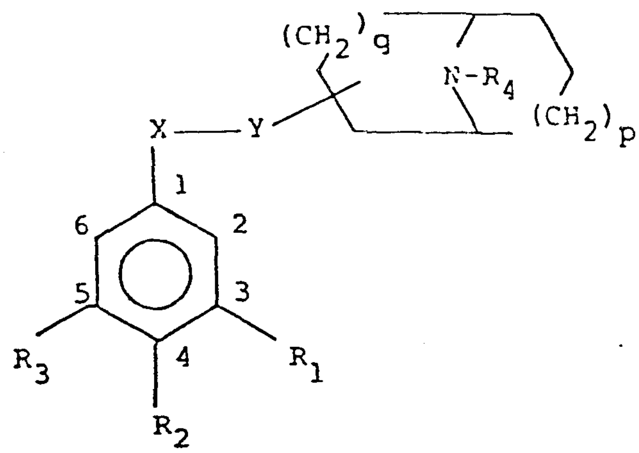

(I)

wherein

one of X and Y is CO and the other is NH;

p and q each independently are 0 to 2;

$R_1$, $R_2$ and $R_3$ are each independently hydrogen, $C_{1-6}$ alkyl, hydroxy, $C_{1-6}$ alkoxy, $C_{1-6}$ alkylthio or halo; or any two on adjacent carbon atoms together are $C_{1-2}$ alkylenedioxy and the third is hydrogen, $C_{1-6}$ alkyl, $C_{1-6}$ alkoxy or halo; and

$R_4$ is $C_{1-7}$ alkyl, $C_{3-8}$ cycloalkyl, $C_{3-8}$ cycloalkyl-$C_{1-2}$ alkyl, or a group $(CH_2)_tR_5$ where t is 1 or 2 and $R_5$ is thienyl or furyl optionally substituted by one or two substituents selected from $C_{1-6}$ alkyl, $C_{1-6}$ alkoxy, trifluoromethyl or halogen, or is phenyl optionally substituted by

one or two substituents selected from $C_{1-4}$ alkoxy, trifluoromethyl, halogen, nitro, carboxy, esterified carboxy, and $C_{1-4}$ alkyl optionally substituted by hydroxy, $C_{1-4}$ alkoxy, carboxy, esterified carboxy or in vivo hydrolysable acyloxy.

2. A compound according to claim 1 wherein X is CO and Y is NH.

3. A compound according to claim 1 or 2 wherein p and q are independently O or 1.

4. A compound according to claim 1,2 or 3 wherein the group Y and the heterobicycle nitrogen atom are separated by 3 carbon atoms.

5. A compound according to claim 1 of formula (II)

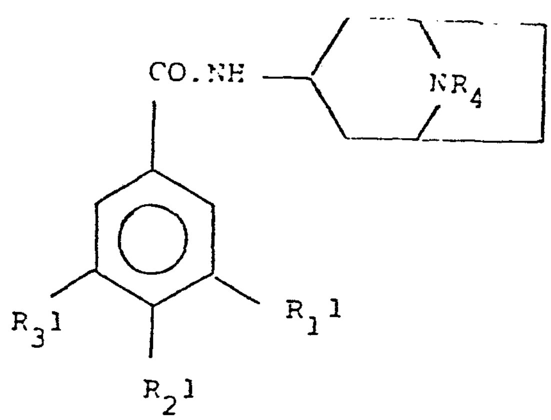

(II)

wherein $R_1{}^1$ is halo or $C_{1-6}$ alkoxy;
$R_2{}^1$ is hydrogen or $C_{1-6}$ alkoxy;
$R_3{}^1$ is halo or $C_{1-6}$ alkoxy; and
$R_4$ is as defined in claim 1.

6. A compound according to claim 5 wherein $R_1{}^1$ is chloro, $R_2{}^1$ is hydrogen or chloro and $R_3{}^1$ is hydrogen or chloro.

7. A compound according to any one of claims 1 to 6 wherein R4 is $C_{1-7}$ alkyl.

8. 3,5-Dichloro-N-[3β-(8-(2,2-dimethylpropyl) -8-azabicyclo (3.2.1)octyl)]benzamide,

3,5-dichloro-N-[3β-(8-methyl-8-azabicyclo (3.2.1)octyl)]benzamide,

3,5-dichloro-N-[3β-(8-benzyl-8-azabicyclo (3.2.1)octyl)]benzamide,

3,5-trimethoxy-N-[3β-(8-methyl-8-azabicyclo (3.2.1)octyl)]benzamide,

3,4-dichloro-N-[3β-(8-methyl-8-azabicyclo (3.2.1)octyl)]benzamide or

3,4-dichloro-N-[3β-(8-methyl-8-azabicyclo (3.2.1)octyl)]benzamide.

9. 3,5-Dichloro-N-[3α-(8-methyl-8-azabicyclo (3.2.1)octyl)]benzamide.

10. A process for the preparation of a compound according to claim 1 or a pharmaceutically acceptable salt thereof, or a pharmaceutically acceptable solvate of any of the foregoing, which process comprises reacting a compound of formula (III):

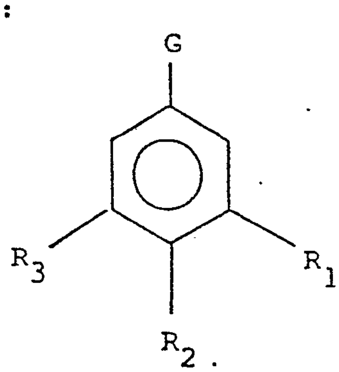

(III)

with a compound of formula (IV):

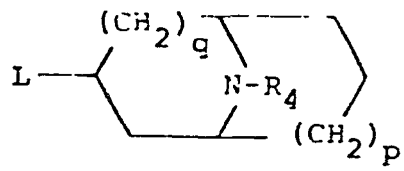

(IV)

wherein

one of G and L is $COQ_1$ where $Q_1$ is a group displaceable by a nucleophile, and the other is $NH_2$; and

thereafter optionally converting any $R_1$, $R_2$, $R_3$ or $R_4$ group to another $R_1$, $R_2$, $R_3$ or $R_4$ group respectively, and optionally forming a pharmaceutically acceptable salt of the resultant compound of formula (I).

11. A pharmaceutical composition comprising a compound according to any one of claims 1 to 9 or a pharmaceutically acceptable salt thereof and a pharmaceutically acceptable carrier.

12. A compound according to any one of claims 1 to 9 or a pharmaceutically acceptable salt thereof for use as an active therapeutic substance.

13. A compound according to any one of claims 1 to 9 for use in the treatment of migraine, cluster headache, trigerminal neuralgia and/or emesis.